# EUROPEAN PATENT SPECIFICATION

(11) **EP 2 691 131 B1**
(45) Date of publication and mention of the grant of the patent: **19.08.2015**
(21) Application number: 12713075.5
(22) Date of filing: 28.03.2012
(51) Int. Cl.: A61M 5/20, A61M 5/50

(54) **INJECTION DEVICE**
INJEKTIONSVORRICHTUNG
DISPOSITIF D'INJECTION

(30) Priority: 30.03.2011 EP 11160445
(43) Date of publication of application: 05.02.2014
(73) Proprietor: Sanofi-Aventis Deutschland GmbH, 65929 Frankfurt am Main (DE)
(72) Inventor: SCHUSTER, Ralf, 65926 Frankfurt am Main (DE)
(86) International application number: PCT/EP2012/055556
(87) International publication number: WO 2012/130901

(56) References cited:
- WO-A1-2006/078400
- WO-A1-2007/128767
- WO-A1-2008/146021
- US-A- 5 536 249
- US-A1- 2007 167 920

## Description

### Technical Field

The invention relates to an injection device for delivering a drug according to the preamble of claim 1. Such an injection device is known from WO 2008/146021 A1.

### Background of the Invention

Many patients, such as diabetics need to be given drugs, e.g. insulin by injection. Particularly older patients may experience difficulties when handling the respective injection devices for administering the drug. Some patients, especially those who are not yet used to injecting themselves may be scared of injection needles. In most cases insulin will be administered shortly after measuring blood glucose by means of a separate measuring device. Patients may thus be excited when performing the injection.

WO 2006/042419 A1 discloses a portable electronic device comprising an outer casing having at least one electrical component and a fluid delivery system therein. The fluid delivery system is integrally defined within at least a portion of the outer casing. The fluid delivery system includes an internal reservoir defined within the outer casing and within which a fluid is contained. The fluid delivery system is operable to dispense the fluid from the portable electronic device. Preferably, the fluid delivery system is an aerosol delivery system which dispenses an aerosol product.

US 2009/0030366 A1 discloses a self-injection system allowing a user to inject a drug from a cartridge carrying unique identification information, into any one of a plurality of injection sites. Tissue at each injection site is associated with at least one injection parameter, such as flow-rate, that is different for each site. A scanner reads the identification information of the cartridge and cooperates with a central processing unit to determine the validity of the drug in order to permit an injection procedure to commence. The central processing unit has a memory for storing the different injection parameters and controls a drive unit for driving fluid from the cartridge and through a needle into the selected tissue, at the injection parameter that is associated with the user selected tissue for the injection.

### Summary of the Invention

It is an object of the present invention to provide an improved injection device.

The object is achieved by an injection device according to claim 1.

Preferred embodiments of the invention are given in the dependent claims.

According to the invention an injection device for delivering a drug comprises a housing arranged to receive a drug container and an electrical energy source, wherein a driver is arranged to displace a dose of the drug from the container through a nozzle for delivering it to a patient under load of a drive spring upon release, wherein an electric motor powered by the electrical energy source is arranged for tensioning the drive spring, wherein control means are arranged for controlling the electric motor so as to tension the drive spring when an injection has been performed. Thus the drive spring is tensioned for the next drug delivery immediately or only after a short time period, for example 5 or 10 seconds. This overcomes the problem that by the actual time of the next injection the battery may be too weak to tension the drive spring. Thus, the user may not be able to perform the injection due to raised energy consumption draining the battery in the mean time or due to a long period until the next injection leading to self-discharge of the battery. Or, the user may only notice at the time of the next injection that the charge level of the battery is too low to perform the injection. However, charging the battery at that time may delay the next injection considerably. Instead, as the drive spring is tensioned immediately, the state of the battery at the time of the next injection is irrelevant. If the charge level of the battery is too low to tension the spring already after an injection, the user is notified immediately. Thus, there is sufficient time to recharge the battery before the next injection. In this way, an increased reliability and usability of the injection device may be provided. Further, the energy required to tension the spring may be higher than the energy for controlling an injection. Controlling an injection shall be understood to comprise activities like sensor readings, user interface communication such as checking key presses and displaying information, calculations, and / or the like. However, it shall not comprise tensioning the spring. Thus, even if after an injection and retensioning of the spring, the energy left in the battery is too low to tension the spring again, there may still be sufficient energy left over for controlling the next injection.

In an alternative embodiment, in case of a low charge condition of the main battery a second highly available battery (e.g. a lithium battery) may be integrated for control of the injection run. For example, this second battery is used only for control functions and not for supplying high current components like the spring tension system.

The main energy source may be a battery, in particular a rechargeable battery.

A blood glucose measuring device may be integrated with the injection device, in particular if the injection device is used for delivering insulin to diabetics. Thus the amount of equipment that the patient has to carry is reduced increasing convenience.

A mobile phone may be integrated with the injection device. This allows for further reducing the amount of equipment to be carried by the patient. The processing resources of the mobile phone may be shared to control the functions of the injection device and the functions of the blood glucose measuring device if applicable. Data and user interfaces of the mobile phone such as visual display, audio output, vibration alarm, keyboard, touch screen, wireless connections such as Bluetooth®, SMS, etc., may be shared to allow interaction with the injection device and with the blood glucose measuring device if applicable. This may be used for compliance monitoring or for reminding the user to administer their dose of drug. The set units to be administered may be displayed. Voice output of data may be used to assist visually impaired users. The user effort to deliver their drug may be reduced to just pushing at least one button or performing at least one gesture on the touch screen. The injection device may remind the user to replace needles or drug containers as required. The injection device may supervise the storage conditions, e.g. the storage temperature of the container and may issue a warning if the storing conditions are out of the specification requiring the container to be replaced. Blood glucose measurements may be stored, processed and graphically displayed. The injection device may recommend the appropriate dosage depending on the blood glucose measurement. The injection device may store and graphically display an injection history comprising the number of units delivered. The stored data may be forwarded to a physician or to a pharma company to allow processing them and/or present them to the patient on a secured website. The injection device may log signals and user inputs so as to allow the physician to recommend an improved administration regime.

A skin contact sensor may be arranged, wherein the control means is linked to the skin contact sensor and arranged to release the drive spring for injection when the skin contact sensor has detected that the injection device is placed against an injection site. The skin contact sensor may be a capacitive sensor.

The needle may be connectable to the container in a manner to be hidden prior to injection, wherein the needle is arranged to be exposed once the skin contact sensor has detected that the injection device has been placed against an injection site. For this purpose the needle may be advanced for insertion into the injection site by a spring and the needle may be arranged to be retracted for hiding the needle by a spring after the end or after an interruption of the injection.

The drive spring may be used for both delivering the drug and advancing the needle.

The control means are arranged for controlling the electric motor so as to tension not only the drive spring but also the springs for advancing and retracting the container when an injection has been performed.

Auxiliary manual means may be arranged for tensioning at least the drive spring and the other springs if applicable. This allows the user to prepare the device for the next injection if the battery state is already insufficient at the end of injection. The injection device may also be arranged to remind the user to recharge the energy source in this case, wherein the drive spring and/or the other springs may be tensioned as soon as the injection device is connected to a charger, when charging is complete or when the state of the energy source is sufficient for tensioning the springs.

The control means may be arranged to set a stop for the driver defining a dose of the drug to be delivered so as to actively ensure a correct drug regime.

Compliance monitoring means may be arranged for supervising a correct administration regime of the drug and a compliant change rate of the needle. Usually the needle has to be replaced on a daily base. Individual needles are packaged in sterile packages and stored in an appropriate mount allowing safe removal of used needles from the injection device and safe connection of new needles to the injection device without subjecting the user to the risk of needle stick injuries.

Means for detecting insertion of a new container may be arranged, wherein venting means are arranged for automatically venting the container after detection of a new container. The user may be asked to confirm the venting in a dialog before the actual venting is performed.

A turbidity sensor may be arranged in the injection device for detecting turbidity of the drug, wherein a warning may be issued if the turbidity exceeds a set value or if the drug is insufficiently mixed inviting the user to mix the drug, e.g. by shaking the device. In addition to this or alternatively, an acceleration sensor may be arranged in the injection device so that an acceleration for mixing the drug is signalled to the micro-processor. Thus, the micro-processor may determine, that the device has been shaken well to mix the drug. For example, the acceleration sensor indicates an acceleration pattern by which the device was moved. Thus, the processor may determine that the device was moved in opposite directions at least every second. The processor may determine also peak acceleration and check that it is above a pre-defined threshold for each movement. In addition, a minimum number of back and forth movements may need to be performed, or the time of the movements of the device needs to exceed a pre-defined threshold. If at least one of these criteria is met, the processor may determine that the device has been shaken sufficiently. The device may indicate this by an indicating lamp, for example an LED that flashes or changes colour (e.g. red to green) when the device has been shaken sufficiently.

A tray may be arranged in the housing for storing blood glucose test strips to be used with a blood glucose measuring device, e.g. with an integrated blood glucose measurement device or with an external one. Preferably the tray is dimensioned to allow storage of the daily requirement of test strips. A mechanism may be arranged to allow easy and individual removal of the test strips. Test strips from a roll or as a segment on a disk may alternatively be used.

The driver may be arranged for pushing a stopper in the container. Alternatively the driver may be arranged as a pump arranged between the container and a needle. In this case the drive spring may be arranged as a torsion spring for rotating the pump. In most cases the drive spring as well as the other springs if applicable may be helical compression springs.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawing which are given by way of illustration only, and thus, are not restrictive of the present invention, and wherein:

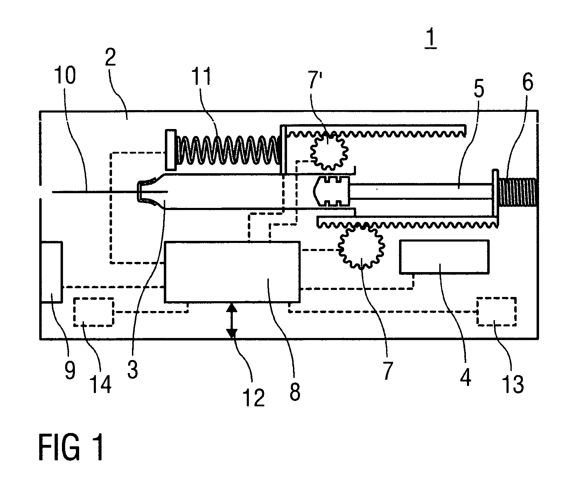

- Figure 1: is a schematic view of an injection device.

### Detailed Description of Preferred Embodiments

Figure 1 shows an injection device 1 for delivering a drug, e.g. insulin. The injection device 1 may be integrated with a blood glucose measuring device and a mobile phone. The injection device 1 comprises a housing 2 receiving a drug container 3 and an electrical energy source 4. A driver 5 is arranged to displace a dose of drug from the container 3 under load of a drive spring 6 upon release. An electric motor 7 powered by the electrical energy source 4 is arranged for tensioning the drive spring 6. Control means 8 are arranged for controlling the electric motor 7 so as to tension the drive spring 6 when an injection has been performed. A skin contact sensor 9 is arranged for detecting whether or not the device is placed against an injection site. An injection needle 10 is connected to the container 3. The injection needle 10 may initially be hidden inside the housing 2.

If the skin contact sensor 9 signals contact to the injection site to the control means 8 the control means 8 exposes the needle 10, e.g. by releasing a spring 11 for advancing the container 3 with the needle 10. The injection is then automatically performed by the control means 8 releasing the drive spring 6. Hence, the driver 5 is operated by the drive spring 6 so as to displace the drug from the container 3 through the injection needle 10 into the injection site, e.g. a patient's skin. The skin contact sensor 9 may supervise skin contact during the injection in a manner interrupting the injection as soon as the injection device 1 is removed from the injection site. In this case or after delivery of the full dose the needle 10 and the container 3 are retracted by the control means releasing a spring, which may be a separate spring or the same spring 11 as the one for advancing the needle 10. This may be achieved by appropriately switching the end of the spring 11 grounded in the housing and the end coupled to the container 3. After the end of injection the control means 8 control the electric motor 7 so as to tension the drive spring 6 and the other spring 11. Thus the springs 6, 11 are tensioned for the next drug delivery immediately.

The energy source 4 may be a battery, in particular a rechargeable battery.

The control means 8 may be integrated in a processor of the mobile phone sharing the processing resources of the mobile phone to control the functions of the injection device 1 and the functions of the blood glucose measuring device. Data and user interfaces 12 of the mobile phone such as visual display, audio output, vibration alarm, keyboard, touch screen, wireless connections such as Bluetooth®, SMS, etc., may be shared to allow interaction with the injection device 1 and with the blood glucose measuring device if applicable. This may be used for compliance monitoring or for reminding the user to administer their dose of drug. The set units to be administered may be displayed. Voice output of data may be used to assist visually impaired users. The injection device 1 may remind the user to replace needles or drug containers 3 as required. The injection device 1 may supervise the storage conditions, e.g. the storage temperature of the container 3 and may issue a warning if the storing conditions are out of the specification requiring the container 3 to be replaced. Blood glucose measurements may be stored, processed and graphically displayed. The injection device 1 may recommend the appropriate dosage depending on the blood glucose measurement. The injection device 1 may store and graphically display an injection history comprising the number of units delivered. The stored data may be forwarded to a physician, a health care professional, to a database, for example of a hospital, a university, or of a pharma company to allow processing and/or present the data to the patient, the physician, the health care professional or any other interested party which has the permission to see the patient related data on a secured website. The injection device 1 may log signals and user inputs so as to allow the physician to recommend an improved administration regime.

The drive spring 6 may be used for both delivering the drug and advancing the needle 10. The drive spring 6 may also perform the task of retracting the container 3 and needle 10 after injection. This may be achieved by electromechanical means controlled by the control means 8 appropriately coupling the ends of the drive spring 6 to the housing 2, the container 3 or the driver 5.

The control means 8 are arranged for controlling the electric motor 7 or a number of electric motors 7, 7' so as to tension not only the drive spring 6 but also the springs 11 for advancing and retracting the container 3 when an injection has been performed.

Auxiliary manual means may be arranged for tensioning at least the drive spring 6 and the other springs 11 if applicable. The injection device 1 may be arranged to remind the user to recharge the energy source 4 if the state of the energy source 4 does not allow to immediately tension the springs 6, 11. The drive spring 6 and/or the other springs 11 may be tensioned as soon as a charger is connected, when the charging is complete or when the state of the energy source 4 is sufficient for tensioning the springs during recharge.

The control means 8 may be arranged to set a stop for the driver 5 defining a dose of the drug to be delivered so as to actively ensure a correct drug regime. The dose of the drug may be set by the patient, the health care professional or the physician. The dose of the drug may vary for each injection or may be fixed, and the stop for the driver 5 may be set accordingly for each injection.

Compliance monitoring means may be arranged for supervising a correct administration regime of the drug and a compliant change rate of the needle 10.

Means for detecting insertion of a new container 3 may be arranged, e.g. based on a coding such as RFID or a bar code. Venting means may be arranged for automatically venting the container 3 and the needle 10 after detection of a new container 3. The venting may be performed by appropriately releasing the drive spring 6 and stopping it in time to prevent leakage of the drug from the needle 10. The user may be asked to confirm the venting in a dialog before the actual venting is performed.

A turbidity sensor may be arranged in the injection device 1 for detecting turbidity of the drug, wherein a warning may be issued if the turbidity exceeds a set value or if the drug is insufficiently mixed inviting the user to mix the drug, e.g. by shaking the device. In addition to this or alternatively an acceleration sensor 14 may be arranged in the injection device for control, that the device has been shaken well to mix the drug.

A tray may be arranged in the housing for storing blood glucose test strips to be used with a blood glucose measuring device, e.g. with an integrated blood glucose measurement device or with an external one. Preferably the tray is dimensioned to allow storage of the daily requirement of test strips. A mechanism may be arranged to allow easy and individual removal of the test strips. Test strips from a roll or as a segment on a disk may alternatively be used.

The driver 5 in the illustrated embodiment is arranged for pushing a stopper in the container 3. Alternatively the driver 5 may be arranged as a pump arranged between the container 3 and a needle 10. In this case the drive spring 6 may be arranged as a torsion spring for rotating the pump. In most cases the drive spring 6 as well as the other springs 11 if applicable may be helical compression springs.

Damping means may be arranged for damping motion of the needle 10 and container 3 during advancing them for needle insertion and/or during retraction.

In case of a low charge condition of the main electrical energy source 4 a second highly available battery 13 (e.g. a lithium battery) may be integrated for control of the injection run. For example, this second battery 13 is used only for control functions and not for supplying high current components like the spring tension system.

### List of References

- 1: injection device
- 2: housing
- 3: drug container
- 4: electrical energy source
- 5: driver
- 6: drive spring
- 7, 7': electric motor
- 8: control means
- 9: skin contact sensor
- 10: injection needle
- 11: spring
- 12: interface
- 13: highly available battery
- 14: acceleration sensor

## Claims

1. Injection device (1) for delivering a drug, comprising a housing (2) arranged to receive a drug container (3) and an electrical energy source (4) being a battery, wherein a driver (5) is arranged to displace a dose of the drug from the container (3) under force of a drive spring (6) upon release irrespective of a state of the battery (4), wherein an electric motor (7) powered by the electrical energy source (4) is arranged for tensioning the drive spring (6), **characterised in that** control means (8) are arranged for controlling the electric motor (7) so as to tension the drive spring (6) immediately or after a short time period when an injection has been performed.

2. Injection device (1) according to claim 1, arranged to notify the user immediately, if a charge level of the battery (4) is too low to tension the spring (6) after the injection.

3. Injection device (1) according to one of the claims 1 or 2, **characterized in that** the battery (4) is a rechargeable battery.

4. Injection device (1) according to claims 2 and 3, arranged to remind the user to recharge the battery (4) if the charge level of the battery (4) is too low to tension the spring (6) after the injection.

5. Injection device (1) according to one of the preceding claims, **characterized in that** auxiliary manual means are arranged for tensioning at least the drive spring (6).

6. Injection device (1) according to one of the claims 4 or 5, arranged to tension the drive spring (6) as soon as a charger is connected or when the charging is complete or when the state of the battery (4) is sufficient for tensioning the spring (6) during recharge.

7. Injection device (1) according to one of the previous claims, **characterized in that** the driver (5) is arranged for pushing a stopper in the container (3).

8. Injection device (1) according to one of the claims 1 to 6, **characterized in that** the driver (5) is arranged as a pump arranged between the container (3) and a needle (10).

9. Injection device (1) according to one of the claims 2 to 8, **characterized in that** the control means (8) is arranged to determine the charge level of the battery (4).

10. Injection device (1) according to one of the previous claims, **characterized in that** a mobile phone is integrated with the injection device (1).

11. Injection device (1) according to claim 10, **characterized in that** a blood glucose measuring device is integrated with the injection device (1).

12. Injection device (1) according to claim 11, **characterized in that** a data interface or user interface of the mobile phone is arranged to notify or remind the user if the charge level of the battery (4) is too low to tension the spring (6) after the injection.

13. Injection device (1) according to one of the preceding claims, **characterized in that** a skin contact sensor (9) is arranged, wherein the control means (8) is linked to the skin contact sensor (9) and arranged to release the drive spring (6) for injection when the skin contact sensor (9) has detected that the injection device (1) is placed against an injection site.

14. Injection device (1) according to claim 13, **characterized in that** an injection needle (10) is connectable to the container (3) in a manner to be hidden inside the housing (2) prior to injection, wherein the needle (10) is arranged to be exposed for insertion into the injection site by a spring (11) when the skin contact sensor (9) has detected that the injection device (1) is placed against the injection site and wherein the needle (10) is arranged to be retracted for hiding the needle (10) after the end or after an interruption of the injection.

15. Injection device (1) according to claim 14, **characterized in that** the spring (11) for advancing the needle is the drive spring (6).

## Patentansprüche

1. Injektionsvorrichtung (1) zur Abgabe eines Arzneimittels, umfassend ein Gehäuse (2), das zum Aufnehmen eines Medikamentenbehälters (3) und einer elektrischen Energiequelle (4), die eine Batterie ist, eingerichtet ist, wobei ein Antrieb (5) eingerichtet ist, unabhängig von einem Zustand der Batterie (4) eine Dosis des Arzneimittels aus dem Behälter (3) unter der Triebkraft einer Antriebsfeder (6) bei Freigabe zu verdrängen, wobei ein Elektromotor (7), angetrieben durch die elektrische Energiequelle (4) zum Spannen der Antriebsfeder (6) eingerichtet ist, **dadurch gekennzeichnet, dass** Steuermittel (8) zur Steuerung des Elektromotors (7) eingerichtet sind, um die Antriebsfeder (6) sofort oder nach einer kurzen Zeitspanne zu spannen, wenn eine Injektion durchgeführt worden ist.

2. Injektionsvorrichtung (1) gemäß Anspruch 1, die eingerichtet ist, den Benutzer umgehend zu benachrichtigen, wenn ein Ladezustand der Batterie (4) zu gering ist, um die Feder (6) nach der Injektion zu spannen.

3. Injektionsvorrichtung (1) gemäß einem der Ansprüche 1 oder 2, **dadurch gekennzeichnet, dass** die Batterie (4) eine wiederaufladbare Batterie ist.

4. Injektionsvorrichtung (1) gemäß Anspruch 2 und 3, die eingerichtet ist, den Benutzer daran zu erinnern, die Batterie aufzuladen (4), wenn der Ladezustand der Batterie (4) zu gering ist, um die Feder (6) nach der Injektion zu spannen.

5. Injektionsvorrichtung (1) gemäß einem der vorhergehenden Ansprüche, **dadurch gekennzeichnet, dass** manuelle Hilfsmittel zum Spannen zumindest der Antriebsfeder (6) eingerichtet sind.

6. Injektionsvorrichtung (1) gemäß einem der Ansprüche 4 oder 5, eingerichtet, die Antriebsfeder (6) zu spannen, sobald ein Ladegerät angeschlossen wird oder wenn der Ladevorgang abgeschlossen ist oder wenn der Ladezustand der Batterie (4) zum Spannen der Feder (6) während der Wiederaufladung ausreichend ist.

7. Injektionsvorrichtung (1) gemäß einem der vorhergehenden Ansprüche, **dadurch gekennzeichnet, dass** der Antrieb (5) zum Einschieben eines Stopfens in den Behälter (3) eingerichtet ist.

8. Injektionsvorrichtung (1) gemäß einem der Ansprüche 1 bis 6, **dadurch gekennzeichnet, dass** der Antrieb (5) als eine Pumpe ausgebildet ist, die zwischen dem Behälter (3) und einer Nadel (10) angeordnet ist.

9. Injektionsvorrichtung (1) gemäß einem der Ansprüche 2 bis 8, **dadurch gekennzeichnet, dass** die Steuermittel (8) eingerichtet sind, den Ladezustand der Batterie (4) zu bestimmen.

10. Injektionsvorrichtung (1) gemäß einem der vorhergehenden Ansprüche, **dadurch gekennzeichnet, dass** ein Mobiltelefon in der Injektionsvorrichtung (1) integriert ist.

11. Injektionsvorrichtung (1) gemäß Anspruch 10, **dadurch gekennzeichnet, dass** eine Blutzuckermessvorrichtung in der Injektionsvorrichtung (1) integriert ist.

12. Injektionsvorrichtung (1) gemäß Anspruch 11, **dadurch gekennzeichnet, dass** eine Datenschnittstelle oder Benutzerschnittstelle des Mobiltelefons eingerichtet ist, den Benutzer zu informieren oder zu erinnern, wenn der Ladezustand der Batterie (4) zu gering ist, um die Feder (6) nach der Injektion zu spannen.

13. Injektionsvorrichtung (1) gemäß einem der vorhergehenden Ansprüche, **dadurch gekennzeichnet, dass** ein Hautkontaktsensor (9) angeordnet ist, wobei die Steuermittel (8) mit dem Hautkontaktsensor (9) verbunden und eingerichtet sind, die Antriebsfeder (6) zur Injektion freizugeben, wenn der Hautkontaktsensor (9) detektiert hat, dass die Injektionsvorrichtung (1) auf einer Injektionsstelle platziert ist.

14. Injektionsvorrichtung (1) gemäß Anspruch 13, **dadurch gekennzeichnet, dass** eine Injektionsnadel (10) mit dem Behälter (3) so verbindbar ist, dass sie im Inneren des Gehäuses (2) vor der Injektion verborgen ist, wobei die Nadel (10) eingerichtet ist, zum Einführen in die Injektionsstelle durch eine Feder (11) freigesetzt zu werden, wenn der Hautkontaktsensor (9) detektiert hat, dass die Injektionsvorrichtung (1) auf der Injektionsstelle platziert ist und wobei die Nadel (10) eingerichtet ist, zum Verbergen der Nadel (10) nach dem Ende oder nach einer Unterbrechung der Injektion zurückgezogen zu werden.

15. Injektionsvorrichtung (1) gemäß Anspruch 14, **dadurch gekennzeichnet, dass** die Feder (11) zum Vorschieben der Nadel die Antriebsfeder (6) ist.

## Revendications

1. Dispositif d'injection (1) pour l'administration d'un médicament, comprenant un boîtier (2) prévu pour recevoir un récipient de médicament (3) et une source d'énergie électrique (4) constituée par une pile, un dispositif d'entraînement (5) étant prévu pour déplacer une dose du médicament depuis le récipient (3) par la force d'un ressort d'entraînement (6) lors de sa libération indépendamment d'un état de la pile (4), un moteur électrique (7) alimenté par la source d'énergie électrique (4) étant prévu pour tendre le ressort d'entraînement (6), **caractérisé en ce que** des moyens de commande (8) sont prévus pour commander le moteur électrique (7) de manière à tendre le ressort d'entraînement (6) immédiatement à la suite d'une injection ou un court moment après celle-ci.

2. Dispositif d'injection (1) selon la revendication 1, prévu pour indiquer immédiatement à l'utilisateur si un niveau de charge de la pile (4) est trop faible pour tendre le ressort (6) après l'injection.

3. Dispositif d'injection (1) selon la revendication 1 ou 2, **caractérisé en ce que** la pile (4) est une pile rechargeable.

4. Dispositif d'injection (1) selon les revendications 2 et 3, prévu pour rappeler à l'utilisateur de recharger la pile (4) si le niveau de charge de la pile (4) est trop faible pour tendre le ressort (6) après l'injection.

5. Dispositif d'injection (1) selon l'une quelconque des revendications précédentes, **caractérisé en ce que** des moyens manuels auxiliaires sont prévus pour tendre au moins le ressort d'entraînement (6).

6. Dispositif d'injection (1) selon l'une quelconque des revendications 4 et 5, prévu pour tendre le ressort d'entraînement (6) dès qu'un chargeur est connecté ou lorsque la charge est terminée ou lorsque l'état de charge de la pile (4) est suffisant pour tendre le ressort (6) lors de la recharge.

7. Dispositif d'injection (1) selon l'une quelconque des revendications précédentes, **caractérisé en ce que** le dispositif d'entraînement (5) est prévu pour pousser un bouchon dans le récipient (3).

8. Dispositif d'injection (1) selon l'une quelconque des revendications 1 à 6, **caractérisé en ce que** le dispositif d'entraînement (5) est prévu sous forme de pompe disposée entre le récipient (3) et une aiguille (10).

9. Dispositif d'injection (1) selon l'une quelconque des revendications 2 à 8, **caractérisé en ce que** les moyens de commande (8) sont prévus pour déterminer le niveau de charge de la pile (4).

10. Dispositif d'injection (1) selon l'une quelconque des revendications précédentes, **caractérisé en ce qu'**un téléphone portable est intégré au dispositif d'injection (1).

11. Dispositif d'injection (1) selon la revendication 10, **caractérisé en ce qu'**un dispositif de mesure du taux de glucose dans le sang est intégré dans le dispositif d'injection (1).

12. Dispositif d'injection (1) selon la revendication 11, **caractérisé en ce qu'**une interface données ou une interface utilisateur du téléphone portable est prévue pour indiquer ou rappeler à l'utilisateur le cas échéant que le niveau de charge de la pile (4) est trop faible pour tendre le ressort (6) après l'injection.

13. Dispositif d'injection (1) selon l'une quelconque des revendications précédentes, **caractérisé en ce qu'**un capteur de contact avec la peau (9) est prévu, les moyens de commande (8) étant reliés au capteur de contact avec la peau (9) et étant prévus pour libérer le ressort d'entraînement (6) en vue de l'injection lorsque le capteur de contact avec la peau (9) a détecté que le dispositif d'injection (1) était placé contre un site d'injection.

14. Dispositif d'injection (1) selon la revendication 13, **caractérisé en ce qu'**une aiguille d'injection (10) peut être connectée au récipient (3) de manière à être dissimulée à l'intérieur du boîtier (2) avant l'injection, l'aiguille (10) étant prévue pour être exposée en vue de l'insertion dans le site d'injection par un ressort (11) lorsque le capteur de contact avec la peau (9) a détecté que le dispositif d'injection (1) était placé contre le site d'injection et l'aiguille (10) étant prévue pour être rentrée afin de dissimuler l'aiguille (10) après la fin de l'injection ou après une interruption de l'injection.

15. Dispositif d'injection (1) selon la revendication 14, **caractérisé en ce que** le ressort (11) pour faire avancer l'aiguille est le ressort d'entraînement (6).
